(19) Europäisches Patentamt
European Patent Office
Office européen des brevets

(11) **EP 2 032 977 B1**

(12) **FASCICULE DE BREVET EUROPEEN**

(45) Date de publication et mention
de la délivrance du brevet:
**13.05.2015 Bulletin 2015/20**

(21) Numéro de dépôt: **07765950.6**

(22) Date de dépôt: **24.05.2007**

(51) Int Cl.:
***G01N 27/48*** *(2006.01)*

(86) Numéro de dépôt international:
**PCT/FR2007/000874**

(87) Numéro de publication internationale:
**WO 2007/138180 (06.12.2007 Gazette 2007/49)**

(54) **PROCEDE, DISPOSITIF ET SYSTEME DE MICROANALYSE D'IONS**

VERFAHREN, VORRICHTUNG UND SYSTEM ZUR MIKROANALYSE VON IONEN

METHOD, DEVICE AND SYSTEM FOR THE MICROANALYSIS OF IONS

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **31.05.2006 FR 0604865**

(43) Date de publication de la demande:
**11.03.2009 Bulletin 2009/11**

(73) Titulaire: **Commissariat à l'Énergie Atomique et aux Énergies Alternatives
75015 Paris (FR)**

(72) Inventeur: **MARCHAND, Gilles
38119 Pierre-Chatel (FR)**

(74) Mandataire: **Reboussin, Yohann Mickaël Noël et al
Cabinet Orès
36, rue de St Pétersbourg
75008 Paris (FR)**

(56) Documents cités:
- BUZZEO M C ET AL: "Non-Holoaluminate Room-Temperature Ionic liquids in Electrochemistry-A Review" CHEMPHYSCHEM - A EUROPEAN JOURNAL OF CHEMICAL PHYSICS & PHYSICAL CHEMISTRY, WILEY VCH, WEINHEIM, DE, vol. 5, no. 8, 10 août 2004 (2004-08-10), pages 1106-1120, XP002399493 ISSN: 1439-4235
- LIU J-F ET AL: "Ionic liquid-based liquid-phase microextraction, a new sample enrichment procedure for liquid chromatography" JOURNAL OF CHROMATOGRAPHY, ELSEVIER SCIENCE PUBLISHERS B.V. AMSTERDAM, NL, vol. 1026, no. 1-2, 13 février 2004 (2004-02-13), pages 143-147, XP004482722 ISSN: 0021-9673 cité dans la demande
- VISSER A ET AL.: "Taskspecific ionic liquids for the extraction of metal ions from aqueous solutions" CHEMICAL COMMUNICATIONS, no. 1, 2001, pages 135-136, XP002419263
- DUBOIS P. ET AL.: "Ionic liquid droplet as e-microreactor" ANALYTICAL CHEMISTRY, vol. 78, 26 mai 2006 (2006-05-26), pages 4909-4917, XP002419264

**Description**

L'invention porte sur un procédé, un dispositif et un système de microanalyse d'ions. Un tel procédé, dispositif et système permet la détection et/ou le dosage d'ions en solution, par exemple d'ions métalliques dans l'eau.

Le dosage des ions des métaux lourds (Ni, Cr, Pb, etc.) dans l'eau est une nécessité sur le plan sanitaire et environnemental. En effet, sur les 41 éléments correspondant à cette définition, 21 sont toxiques pour l'homme et l'environnement. Cependant, la détection et/ou le dosage directs de ces éléments dans l'eau peuvent s'avérer très problématiques en raison de leur présence souvent sous forme de traces et des limitations des techniques actuelles en terme de sensibilité. Ainsi, pour faciliter leur analyse, une phase d'extraction dans des solvants organiques de plus faible volume, afin de concentrer les espèces, s'avère souvent nécessaire. L'extraction de ces métaux s'effectue généralement grâce à l'utilisation de molécules complexantes ou chélatantes en solution dans ces solvants.

Il est avantageux que l'extraction et l'analyse des ions soient effectuées sur des échantillons de faible volume, typiquement moins d'un millilitre, notamment afin de réduire la quantité de solvant nécessaire. En particulier, il est souhaitable de pouvoir effectuer ces opérations dans un dispositif de microanalyse, dit aussi « laboratoire sur une puce ». L'article « Continuous-Flow Chemical Processing on a Microchip by Combining Microunit Opérations and a Multiphase Flow Network » de Manabu Tokeshi et al. Anal. Chem. 2002, 74, 1565 - 1571 décrit un système microfluidique permettant l'extraction et l'analyse d'ions Co(II) par complexation dans un dispositif microfluidique. Le solvant utilisé pour l'extraction est le m-Xylène et l'agent complexant le 2-nitroso-1-naphtol. Avec un tel solvant, l'extraction est thermodynamiquement défavorisée, donc son rendement est faible. En outre, la volatilité du solvant ne permet pas son utilisation en faible volume sans l'utilisation d'un capotage, ce qui rend le dispositif d'analyse plus complexe et constitue un obstacle à la mise en oeuvre aisée du procédé en parallèle sur un grand nombre échantillons. De tels problèmes se rencontrent avec la plupart des solvants habituels.

Les liquides ioniques à température ambiante (ci après, liquides ioniques) sont des solvants connus qui présentent des propriétés intéressantes, notamment une tension de vapeur négligeable et la capacité de solubiliser la plupart des molécules organiques ou inorganiques. En fonction de l'anion ou du cation utilisé, les liquides ioniques peuvent être miscibles ou immiscibles avec l'eau.

Les articles suivants ont montré la possibilité d'utiliser des liquides ioniques comme solvants pour l'extraction d'ions métalliques par des éthers couronne, avec des coefficients de partage plus grands qu'en cas d'utilisation d'un solvant conventionnel (« solvant organique volatil ») :

Sheng Dai et al. « Solvent extraction of strontium nitrate by a crown ether using room-temperature ionic liquids », J. Chem. Soc. Dalton Trans. 1999, 1201-1202 ;
Huimin Luo et al. « Extraction of Cesium Ions from Aqueous Solutions Using Calix[4]arene-bis(tert-octylbenzo-crown-6) in Ionic Liquids », Anal. Chem. 2004, 76, 3078-3083.

Les techniques décrites dans ces articles nécessitent cependant l'utilisation de quantités relativement importantes de liquides ioniques (plusieurs millilitres), et de quantités encore plus importantes de la solution aqueuse à analyser. En outre, dans ces techniques l'analyse est faite de manière délocalisée par rapport à l'extraction, ce qui rend nécessaires des manipulations complexes.

L'article de Jing-fu Liu et al « Ionic liquid-based liquid-phase microextraction, a new sample enrichment procédure for liquid chromatography », Journal of Chromatography A, 1026 (2004), 143-147 décrit une technique d'extraction d'ions métalliques utilisant un faible volume (microlitres) d'un liquide ionique suspendu sous forme d'une gouttelette dans un récipient contenant une solution aqueuse à analyser, le dosage des ions étant ensuite réalisé par chromatographie. Cette technique est complexe et requiert un grand nombre de manipulations longues et délicates qui sont difficiles à automatiser.

L'article de M.Buzzeo et al. «Non-Holo aluminat Room-Temperature Ionic Liquids in Electrochemistry - A Review » CHEM PHYSCHEM, Wiley, Vol. 5, No. 8, fait état de plusieurs techniques d'analyse électrochimique exploitant des liquides ioniques.

Un but de l'invention est de proposer un procédé, un dispositif et un système de microanalyse d'ions ne présentant pas, ou présentant en forme atténuée, au moins certains des inconvénients ci-dessus.

Conformément à l'invention, l'extraction et l'analyse des ions sont effectués dans une même cavité contenant un faible volume d'un liquide ionique et des moyens d'analyse d'ions au contact de ce liquide ionique ; le volume du liquide ionique doit être inférieur au volume interne de la cavité, de manière à permettre l'introduction dans cette dernière d'un volume de la solution à analyser. Une « cavité » au sens de l'invention peut être constituée, par exemple, par un canal ou sillon dans une puce fluidique, ou bien par un puits.

Grâce à la très faible tension de vapeur des liquides ioniques, qui ne s'évaporent pratiquement pas, la cavité contenant le liquide ionique constitue un dispositif qui peut être conservé prêt à l'emploi pendant une longue période, et cela sans besoin de prévoir un capotage.

Comme les opérations d'extraction et d'analyse des ions sont effectuées à l'intérieur de la même cavité, un procédé de microanalyse selon l'invention ne nécessite pas de manipulations délicates et peut être mis en oeuvre de manière simple et en grande partie automatique. Plusieurs analyses peuvent être conduites en parallèle grâce à un système de microanalyse constitué par une pluralité de dispositifs selon l'invention, par exemple afin de détecter et/ou doser un même type d'ions dans une pluralité de solutions différentes, ou bien plusieurs types d'ions dans une même solution.

Par ailleurs, la bonne conductivité électrique des liquides ioniques rend possible l'utilisation de techniques électrochimiques pour le dosage des ions extraits, ce qui permet de simplifier encore la structure du dispositif de l'invention et son procédé d'utilisation.

Par « analyse » on entend non seulement une analyse quantitative ou dosage, mais également une simple détection des ions extraits par le liquide ionique.

Plus précisément, un objet de l'invention est un procédé de microanalyse d'ions comportant les étapes suivantes :

- disposer, à l'intérieur d'une cavité ayant un volume interne, un volume d'un liquide ionique qui est inférieur audit volume interne ;
- disposer à l'intérieur de ladite cavité une solution contenant lesdits ions à analyser, le solvant de la solution et le liquide ionique étant choisis de manière à être immiscibles et à permettre un transfert des ions de ladite solution vers ledit liquide ionique ; et
- détecter la présence desdits ions dans ledit liquide ionique à l'aide d'un moyen d'analyse d'au moins un type d'ions en solution dans ledit liquide ionique, à l'état libre ou de complexe, ledit moyen d'analyse étant disposé au contact dudit liquide ionique.

Selon des modes de réalisation particuliers du procédé de l'invention :

- Le procédé peut comporter également une étape d'agitation desdits liquide ionique et solution de manière à favoriser ledit transfert des ions.
- Ladite solution contenant les ions à analyser peut être une solution aqueuse.
- Lesdits ions peuvent être des ions métalliques.
- Ladite étape consistant à détecter la présence desdits ions dans ledit liquide ionique à l'aide dudit moyen d'analyse peut comporter une mesure quantitative de la concentration desdits ions.
- Le liquide ionique peut contenir en solution au moins une molécule ayant une fonction de complexation dudit type d'ions à extraire et analyser.
- Le liquide ionique peut être mélangé à un liquide ionique à tâche spécifique dont au moins un anion ou un cation porte une fonction de complexation dudit type d'ions à extraire et analyser.
- Ladite fonction de complexation peut être une fonction sélective, permettant la complexation d'un seul type d'ions.
- Ledit liquide ionique ou ladite molécule ayant une fonction de complexation peuvent comprendre une fonction sonde dont au moins une propriété physique ou chimique détectable varie en réponse à la complexation des ions à analyser.
- Ledit volume de liquide ionique peut être inférieur à la moitié, de préférence inférieur à un dixième et de manière encore préférée inférieur à un centième du volume de la solution contenant les ions à analyser.
- Le volume interne de ladite cavité peut être inférieur à 1 ml, et de préférence inférieur à 500 µl.
- Ledit volume de liquide ionique peut être inférieur à 10 µl, et de préférence de l'ordre de 1 µl ou moins.
- Ledit liquide ionique peut présenter une densité supérieure à celle de ladite solution.
- Ladite cavité peut présenter un fond et au moins une paroi latérale, ledit volume de liquide ionique étant en quantité juste suffisante pour couvrir ledit fond. Alternativement, le liquide ionique peut être présent en quantité encore plus faible, de manière à former une goutte sur ledit fond, au contact dudit moyen d'analyse.
- Ledit moyen d'analyse d'au moins un type d'ions en solution dans ledit liquide ionique peut être un moyen d'analyse électrochimique comprenant au moins deux et de préférence trois électrodes en contact avec ledit volume de liquide ionique, ladite étape consistant à détecter la présence desdits ions dans ledit liquide ionique à l'aide dudit moyen d'analyse étant effectuée au moyen d'une technique électrochimique, en particulier une mesure par voltampérométrie pulsée différentielle ou par voltampérométrie cyclique.
- Alternativement, ledit moyen d'analyse d'au moins un type d'ions en solution dans ledit liquide ionique peut être un moyen d'analyse optique par spectrophotométrie ou luminescence, ladite étape consistant à

détecter la présence desdits ions dans ledit liquide ionique à l'aide dudit moyen d'analyse étant effectuée au moyen d'une technique optique.

Un autre objet de l'invention est un dispositif pour la mise en oeuvre d'un tel procédé de microanalyse d'ions, comprenant :

- une cavité ayant un volume interne ;
- un volume d'un liquide ionique disposé à l'intérieur de ladite cavité, ledit volume de liquide ionique étant inférieur au volume interne de la cavité ; et
- un moyen d'analyse d'au moins un type d'ions en solution dans ledit liquide ionique à l'état libre ou de complexe, ledit moyen d'analyse étant disposé à l'intérieur de ladite cavité, en contact avec ledit volume de liquide ionique.

Selon des modes de réalisation particuliers du dispositif de l'invention :

- Ladite cavité peut présenter un fond constitué par un substrat de circuit intégré sur lequel sont formées au moins deux, et de préférence au moins trois, électrodes en contact avec ledit volume de liquide ionique et constituant un moyen d'analyse électrochimique d'au moins un type d'ions en solution dans ledit liquide ionique.
- Ladite cavité peut présenter une paroi latérale formée par un puits d'une plaque à puits fixée de manière étanche audit substrat de circuit intégré.

Encore un autre objet de l'invention est un système pour la microanalyse d'ions comportant une pluralité de tels dispositifs, chacun contenant un liquide ionique pour l'extraction sélective d'un type d'ions différent.

Encore un autre objet de l'invention est un système pour la microanalyse d'ions comportant une pluralité de tels dispositifs, chacun contenant un liquide ionique pour l'extraction sélective d'un même type d'ions.

D'autres caractéristiques, détails et avantages de l'invention ressortiront à la lecture de la description faite en référence aux dessins annexés donnés à titre d'exemple et qui représentent, respectivement :

- la figure 1, un schéma de principe d'un dispositif selon l'invention ;
- les figures 2A à 2E, les différentes étapes d'un procédé de microanalyse selon l'invention ;
- les figures 3A et 3B, un dispositif de microanalyse selon un premier mode de réalisation particulier de l'invention ;
- la figure 4, un dispositif de microanalyse selon un deuxième mode de réalisation particulier de l'invention ;
- la figure 5, un dispositif de microanalyse selon un troisième mode de réalisation particulier de l'invention ;
- la figure 6, un système de microanalyse constitué par une pluralité de dispositifs selon un quatrième mode de réalisation particulier de l'invention.

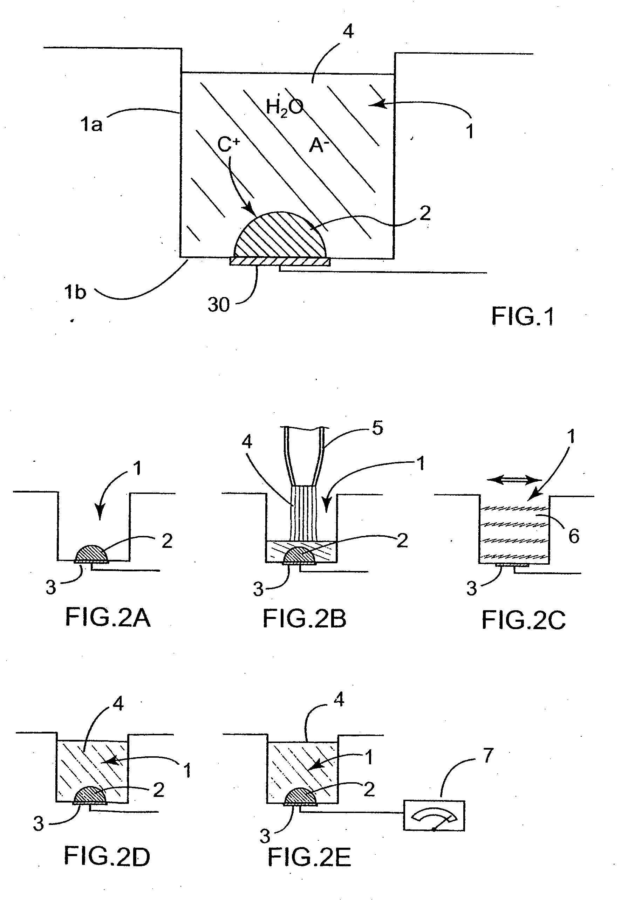

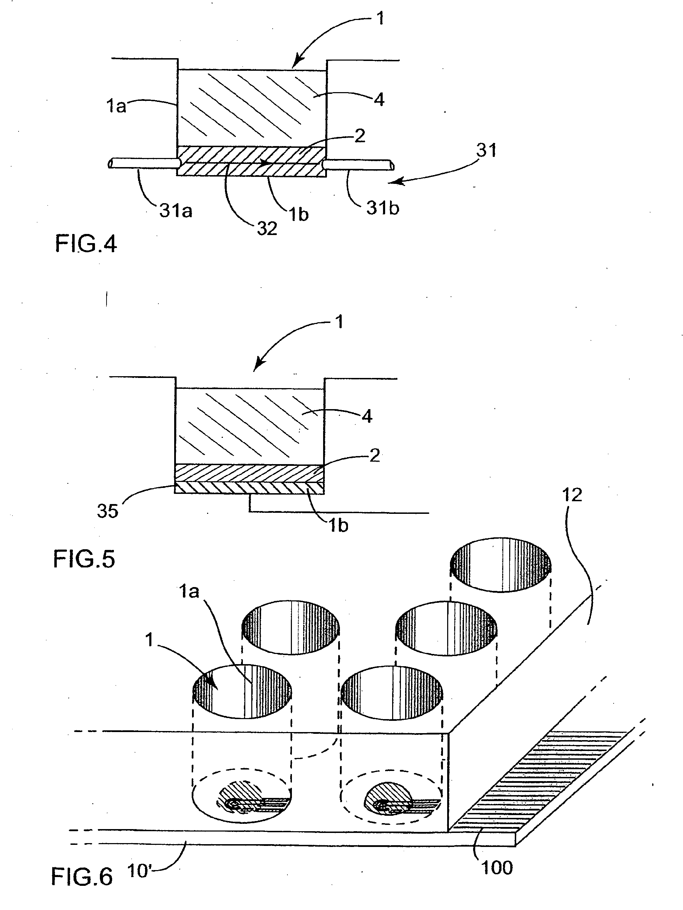

Un dispositif selon l'invention est constitué par une cavité 1, comportant généralement une paroi latérale 1 a et un fond 1 b, ayant un volume interne et contenant un volume prédéfini, inférieur audit volume interne, d'un liquide ionique. Dans le cas de la figure 1, le liquide ionique 2 forme une goutte déposée sur le fond 1 b de la cavité 1. A l'intérieur de la cavité 1, en contact avec la goutte de liquide ionique 2, se trouve un moyen 3 d'analyse (détection et/ou dosage) d'ions. Dans l'exemple de la figure 1, ce moyen 3 est disposé sur le fond 1 b de la cavité 1 et est entièrement recouvert par la goutte de liquide ionique 2 ; en variante, comme montré sur la figure 4, le moyen 3 peut être agencé dans la paroi latérale 1a de ladite cavité. La cavité 1 présente généralement un volume interne inférieur à 1 ml, typiquement de quelques centaines de $\mu$l, par exemple de $500\mu$l. Le volume de la goutte 2 de liquide ionique est sensiblement inférieur, par exemple de quelques $\mu$l, voire de 1 $\mu$l environ. Par « liquide ionique » on entend un sel ou mélange de sels, généralement organiques, qui est liquide à une température comprise entre -100°C et 250°C et plus particulièrement à une température inférieure à 100°C, voire à la température ambiante (ces sels sont connus en littérature anglophone sous l'acronyme de « RTIL » - « Room Temperature Ionic Liquids », liquides ioniques à température ambiante).

La partie du volume interne de la cavité 1 qui n'est pas occupée par la goutte 2 de liquide ionique peut être remplie par une solution à analyser 4, par exemple une solution aqueuse, qui n'est pas considérée comme faisant partie du dispositif. Le liquide ionique 2 étant plus lourd que l'eau (densité généralement de l'ordre de 1,3 - 1,4 g/cm$^3$) et immiscible avec elle, il maintient sa forme de goutte déposée sur le fond 1 b de la cavité et reste en contact avec le moyen d'analyse d'ions. En variante, pour assurer un bon contact entre le liquide ionique 2 et le moyen d'analyse 3 disposé sur le fond de la cavité 1 tout en permettant une concentration élevée des ions à analyser, il peut être avantageux d'utiliser une quantité dudit liquide ionique 2 juste suffisante pour couvrir entièrement le fond 1 b de ladite cavité, au lieu de former une goutte comme dans le cas de la figure 1.

Initialement, la solution aqueuse 4 contient des cations métalliques C$^+$ et des anions A$^-$ ; le liquide ionique 2 présente une affinité élevée avec les cations métalliques et tend donc à les extraire ; l'extraction des ions par le liquide ionique est, en elle-même, favorisée thermodynamiquement, mais elle peut être rendue encore plus rapide et efficace par des réactions de complexation, comme cela sera décrit en détail plus loin. Le volume du liquide ionique 2 étant inférieur ou égal à celui de la solution aqueuse 4, l'extraction peut avoir l'effet de concentrer les cations métalliques C$^+$, ce qui permet leur analyse par le moyen 3 même lorsqu'ils ne sont présents qu'à l'état de traces dans ladite solution.

On comprend que, pour réaliser une extraction des cations métalliques C$^+$, il est nécessaire que le coefficient de partage D, défini comme le rapport à l'équilibre entre les concentrations des ions dans le liquide ionique et dans l'eau, soit suffisamment élevé, par exemple de l'ordre de 10$^3$. Dans un mode de réalisation avantageux de l'invention, l'extraction est assistée par une réaction de complexation, ce qui permet d'atteindre des coefficients de partage dépassant parfois $10^4$.

Une première possibilité de mise en oeuvre de l'invention comporte l'utilisation de liquides ioniques dits « matrices », qui sont chimiquement inertes et ont uniquement un rôle de solvants, dans lesquels sont solubilisées des molécules possédant une fonction de complexation. Des exemples de liquides ioniques matrices adaptés à la mise en oeuvre de l'invention sont (liste non exhaustive) :

- 1-butyl-3-méthylimidazolium hexafluorophosphate (BmimPF6)
- 1-butyl-3-méthylimidazolium bis (trifluorométhylsulfonyl) imide (BmimNTF2)
- 1-éthyl-3-méthylimidazolium hexafluorophosphate (EmimPF6)
- 1-éthyl-3-méthylimidazolium tris (pentafluoroéthyl) trifluorophosphate (EMIMFAP)
- 1-butyl-1-méthylpyrrolidinium tris (pentafluoroéthyl) trifluorophosphate (BMPFAP)
- butyltriméthylammonium bis (trifluorométhylsulfonyl) imide (BtmaNTF2)
- 1-butyl-1-methyl-pyrrolidinium bis (tri-fluoromethylsulfonyl) imide (BMPTF2)
- autres sels d'ammonium, de phosphonium, d'imidazolium, de pyridinium, de guanidinium.

La molécule complexante peut être choisie dans la liste non exhaustive comprenant les éthers couronnes, les cryptands, les podands, les cyclophanes, les calixarènes, les acides carboxyliques, les thiourées, l'urée, les thioéthers, les dérivés borés, les lariats, les rotaxanes, les azines, l'EDTA (acide éthylène-diamine-tétracétique) et les enzymes.

Une autre possibilité consiste à mélanger au liquide ionique matrice un liquide ionique à tâche spécifique, portant sur l'anion, sur le cation ou sur les deux ions constituants une fonction complexante, choisie dans la liste ci-dessus. L'utilisation d'un liquide ionique à tâche spécifique ou d'un sel d'onium à l'état pur se heurte au fait que ces substances ne sont généralement pas liquides à température ambiante.

Qu'elle soit portée par une molécule de soluté ou par l'un des ions constituants un liquide ionique à tâche spécifique mélangé au liquide ionique matrice, la fonction complexante est avantageusement spécifique, c'est-à-dire qu'elle permet d'extraire de manière préférentielle un seul type d'ions.

Dans des modes de réalisation particuliers de l'invention le liquide ionique 2 comporte, généralement greffée à la fonction ou molécule complexante, une fonction « sonde » dont au moins une propriété physique ou chimique détectable (propriétés optiques, électrochimiques, etc.) est influencée par la complexation des ions. La « sonde » permet donc la détection indirecte desdits ions. Dans d'autres modes de réalisation de l'invention, la fonction complexante accomplit également une fonction de sonde.

Des exemples non exhaustifs de sondes sont :

- le ferrocène, le nitrophényl, le couple quinone-hydroquinone et le pyrène, pour une détection électrochimique ;
- le pyrène ou tout fluorophore susceptible d'avoir une réponse différente avant et après complexation d'un ion, pour une détection optique ;
- des enzymes, agissant aussi bien en tant que molécules complexantes sélectives et en tant que sondes biologiques.

Lorsque l'espèce ionique présente une « signature » adaptée, il est également possible de la détecter directement, sans avoir recours à une sonde, notamment par des techniques électrochimiques. La signature permettant la détection peut être une réponse redox : c'est le cas, par exemple, du zinc, du fer, du chlore, du plomb, du cadmium, etc.

On a considéré ici le cas de l'extraction et de la détection et/ou dosage des cations $C^+$. Cependant, l'invention permet également l'extraction et l'analyse des anions $A^-$ de la solution, ou encore des deux types d'ions ; il suffit pour cela d'utiliser une combinaison adaptée du liquide ionique 2 et du moyen d'analyse 3. Par ailleurs, les cations $C^+$ n'ont pas nécessairement à être métalliques : il peut s'agir par exemple de cations phosphonium ou ammonium.

Le tableau ci-après montre des exemples spécifiques de liquides ioniques permettant la détection indirecte, électrochimique ou optique, d'espèces ioniques. La première colonne du tableau contient des ions à détecter ; la deuxième colonne, des liquides ioniques matrices immiscibles avec l'eau ; la troisième colonne, des liquides ioniques à tâches spécifiques portant une fonction sonde et pouvant être mélangés auxdits liquides ioniques matrices pour extraire et détecter les ions de la première colonne ; la quatrième colonne contient des molécules complexantes portant elles aussi une fonction sonde et pouvant être solubilisées dans les liquides ioniques matrices pour extraire et détecter lesdits ions ; la cinquième colonne, enfin, indique la méthode de détection utilisable : électrochimique (E) ou optique (O).

| Ion à détec-ter | Liquide ionique matrice | Liquide ionique à tâche spécifique avec fonction sonde | Molécule complexante avec fonction sonde | Détection |
|---|---|---|---|---|
| Ca$^{2+}$ | BmimPF6 | | | E |

| Hg$^{2+}$ | BmimP F6 | | | E |
| Hg$^{2+}$ | BmimP F6 | - | | E |
| Hg$^{2+}$ | BmimP F6 | | - | E |
| Hg$^{2+}$ | BMPNT F2 | | | O |

7

| | | | | |
|---|---|---|---|---|
| K$^+$ | BMPFAP | – | | E |
| F$^-$ | BMPFAP | – | | E |

Les différentes étapes d'un procédé de microanalyse d'ions selon l'invention sont représentées sur les figures 2A à 2E.

La première étape (figure 2A) consiste à procurer un dispositif selon l'invention, ne contenant pas encore la solution 4 à analyser:

La deuxième étape (figure 2B) consiste en l'introduction, par exemple à l'aide d'une pipette 5, d'une solution 4 à analyser dans la cavité 1 du dispositif de l'invention.

Afin d'accélérer l'extraction, il est avantageux de maximiser la surface de contact entre la solution à analyser 4 et le liquide ionique 2. Pour ce faire, il est opportun de prévoir une troisième étape d'agitation (figure 2C) pour former une émulsion 6 dudit liquide ionique 2 dans la solution aqueuse 4, dans laquelle il est immiscible. L'agitation peut être réalisée, par exemple, en plaçant le dispositif à l'intérieur d'un thermomixeur. La complexation des ions et leur extraction ont lieu principalement au cours de cette phase d'agitation.

La différence de densité entre le liquide ionique 2 et la solution aqueuse 4 entraîne un séparation par décantation entre ces deux phases immiscibles (figure 2D). A ce point, la quasi-totalité des cations métalliques C$^+$ sont passés en solution dans le liquide ionique 2, sous la forme de complexes si un agent de complexation est présent.

Enfin, les moyens 3 d'analyse d'ions, en contact avec le liquide ionique 2, effectuent une détection, et de préférence un dosage, des ions extraits par ledit liquide ionique. Un instrument de mesure 7 permet l'affichage des résultats de cette étape de détection ou dosage.

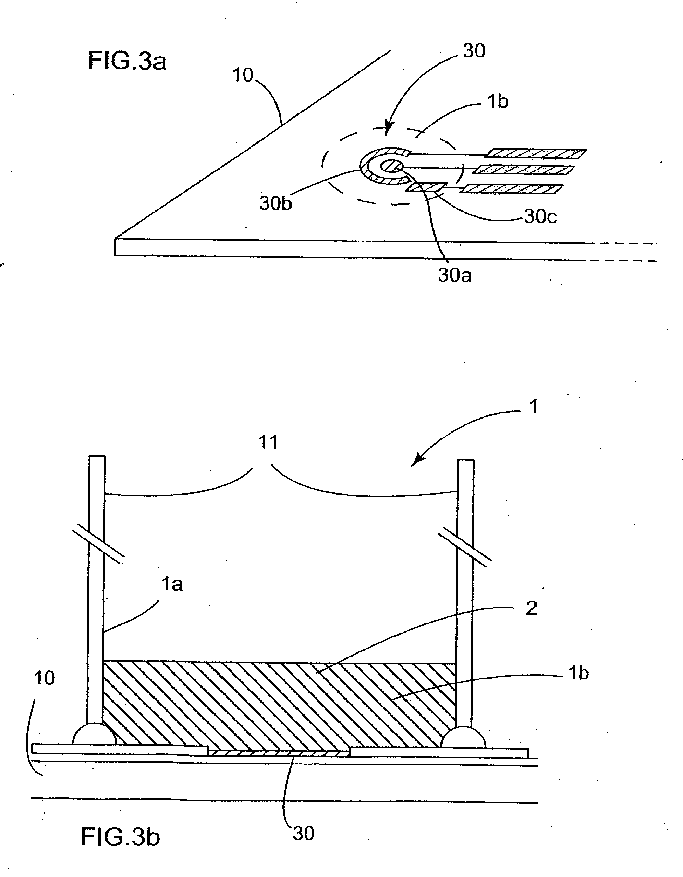

Un exemple détaillé de réalisation d'un dispositif et d'un procédé selon l'invention sera maintenant décrit en référence aux figures 3A et 3B.

Le fond 1 b d'une cavité 1 selon l'invention est réalisé par des techniques microélectroniques de fabrication des circuits intégrés, en partant d'un substrat 10 de silicium dopé n ayant un diamètre de 100 mm et couvert d'une couche de 500nm de SiO$_2$ obtenue par oxydation à 1050°C sous un flux de vapeur d'eau. Une couche de platine Pt de 500 nm d'épaisseur est déposée par pulvérisation sur ce substrat. Ensuite, une couche de résine photosensible est déposée par centrifugation sur la couche de Pt. La couche de résine photosensible est exposée à la lumière à travers un masque photolithographique pour définir un motif 30 de microélectrodes et pistes conductrices, constituant un moyen pour l'analyse électrochimique d'ions. En particulier le motif comprenait une électrode de travail circulaire 30a d'un diamètre de 300 $\mu$m, une contre-électrode 30b en forme de couronne circulaire de 130 $\mu$m de large entourant l'électrode de travail et une électrode de référence 30c de forme rectangulaire de dimensions 50x130 $\mu$m, la distance entre électrodes étant de 70 $\mu$m.

Après enlèvement de la résine photosensible non exposée, le motif est gravé en utilisant un faisceau d'ions d'Argon (« Argon Ion Batch Etch System, Veeco Microtech 801 ») ; après quoi la résine exposée est enlevée à son tour.

Une couche de SiO$_2$ d'une épaisseur de 500 nm est formée par dépôt chimique en phase vapeur assisté par plasma (PECVD - « Plasma Enhanced Chemical Vapor Deposition » en anglais) sur la surface du substrat 10 (dépôt effectué à 300°C par une machine « STS Multiplex » en utilisant un mélange de SiH$_4$ et N$_2$O) et étuvé à 500°C pendant 3 heures sous un flux d'azote.

Une deuxième couche de résine photosensible est déposée sur la couche de SiO$_2$, et elle est exposée de manière à permettre l'ouverture de ladite couche de SiO$_2$ en correspondance des électrodes 30a, 30b et 30c et des points de connexion des pistes conductrices. L'ouverture est effectuée par gravure en utilisant un faisceau d'ions réactifs CHF$_3$ / O$_2$ (machine « Nextral 100 »).

La paroi latérale 1 a délimitant la cavité 1 est constituée par un tube 11 en polyéthylène collé sur le substrat formant le fond 1 b au moyen d'une colle « Vitralit 7105 » polymérisée aux UV. Le volume total de la cavité ainsi obtenue est de 500 $\mu$l.

Un volume d'environ 100 $\mu$l de [bmim][PF$_6$] contenant 2 mg / ml de 1,4-bis(ferrocenyl)-2,3-diaza-1,3-butadiène en tant qu'agent complexant est déposé sur le fond 1 b de la cavité, au contact du système d'électrodes 30. Le 1,4-bis(ferrocenyl)-2,3-diaza-1,3-butadiène a été synthétisé suivant la méthode décrite par Caballero et al., J. Am. Chem. Soc. 2005, 127 (45) pp. 15666-15667.

Une solution aqueuse 4 de 200μl HgCl$_2$ à 10mM est introduite dans la cavité 1 du dispositif ainsi fabriqué. L'extraction est effectuée à température ambiante dans un thermomixeur à 1500 t/min. utilisé comme dispositif d'agitation, pour une durée de 10 min. Ensuite, le système d'électrodes 30 est connecté à un potentiostat pour effectuer une mesure électrochimique par voltampérométrie pulsée différentielle ou par voltampérométrie cyclique. La complexation du mercure par la fonction azine induit une modification de la réponse électrochimique du ferrocène, agissant en tant que sonde électrochimique. La concentration en ions Hg$^{2+}$ dans le liquide ionique 2 est liée au déplacement en potentiels (ΔE) par des abaques ΔE=f([ions ]), où ΔE est la variation du potentiel d'oxydo-réduction de la sonde avant et après complexation et [ions] est la concentration des ions concentrés. Ces abaques dépendent de la nature de la sonde et des ions complexés. Lorsqu'on utilise une technique d'analyse électrochimique directe (sans sonde), on peut se servir de l'équation de Randles-Sevcik :

$$i_p = (2{,}69 \cdot 10^5)n^{3/2} \cdot A \cdot D^{1/2} \cdot C \cdot v^{1/2}$$

où ip est le courant de pic, n le nombre d'électrons de la réaction d'oxydoréduction, A l'aire de la surface de l'électrode de travail, C la concentration des ions en solution à détecter et v la vitesse de balayage. En connaissant le rapport entre les volumes de liquide ionique 2 et de solution aqueuse 4 dans la cavité, et en supposant une extraction complète des ions métalliques, il est possible de calculer leur concentration de départ dans la solution à analyser.

La détection et/ou le dosage des ions métalliques peuvent se faire par des techniques autres qu'électrochimiques, généralement en utilisant une sonde, par exemple par spectrophotométrie, par bioluminescence ou par colorimétrie. En ce qui concerne les techniques électrochimiques, elles peuvent être à deux ou, de préférence, à trois électrodes.

La figure 4 montre un dispositif selon un deuxième mode de réalisation de l'invention, dans lequel le moyen d'analyse d'ions 31 est constitué par deux fibres optiques 31 a et 31 b qui pénètrent dans la cavité 1 à travers sa paroi latérale 1 a ; les surfaces d'extrémité des deux fibres optiques 30a, 30b se font face, de telle manière qu'un rayon lumineux 32 sortant de l'une des deux fibres puisse pénétrer dans l'autre. Dans le cas du dispositif de la figure 4, le liquide ionique 2 n'est pas sous forme d'une goutte, mais remplit partiellement la cavité 1 et touche sa paroi latérale 1 a jusqu'à une hauteur supérieure à celle des surfaces d'extrémité des fibres optiques 31 a, 31b. En conditions de fonctionnement, un faisceau lumineux 32 sort de la fibre optique 31a, traverse le liquide ionique 2 et rentre dans la fibre optique 31b, qui l'amène jusqu'à un spectrophotomètre (non représenté). La complexation d'ions métalliques induit une modification dans le spectre d'absorption ou de fluorescence d'une fonction sonde du liquide ionique (par exemple une sonde à base de pyrène), modification qui est mise en évidence par ledit spectrophotomètre et permet la détection ou le dosage desdits ions.

Les fibres optiques peuvent être remplacées, par exemple, par des guides d'onde planaires intégrées.

La figure 5 montre un dispositif selon un troisième mode de réalisation de l'invention, dans lequel le moyen d'analyse d'ions est constitué par un photodétecteur 35 s'étendant sur la totalité ou sur une partie significative du fond 1b de la cavité 1. Le liquide ionique 2 contient comme molécule complexante une enzyme ne fonctionnant qu'en présence de certains ions et stable dans le liquide ionique choisi. Par exemple, l'alcaline phosphatase et la luciférase ne fonctionnent qu'en présence d'ions Mg$^{2+}$ et leur réponse est fonction de la concentration de ces ions. Le liquide ionique contient également un ou plusieurs substrats enzymatiques et le produit de la réaction enzymatique est luminescent. En présence d'ions Mg$^{2+}$, l'enzyme catalyse la transformation du ou des substrats en un produit luminescent, qui émet des photons qui sont captés par le photodétecteur 35. Pour la luciférase, des substrats adaptés sont la luciférine et l'ATP et pour l'alcaline phosphatase un dérivé de l'adamantyl 1,2-dioxétane aryl phosphate.

En variante, le photodétecteur 35 peut être aménagé sur la paroi latérale 1 a de la cavité 1.

Les dispositifs représentés sur les figures 4 ou 5 peuvent être utilisés, par exemple, pour la détection optique du mercure (ion Hg$^{2+}$). A ce fin, on dépose dans la cavité 1 d'un tel dispositif un volume 2 d'environ 100 μl de BMPNTF2 contenant 5·10$^{-4}$M de 8-hydroxyquinoline benzoate en tant qu'agent complexant et sonde fluorescente, ainsi qu'un volume 4 de 300 μl d'une solution aqueuse de HgCl$_2$ à 10mM. L'extraction peut être effectuée à température ambiante dans un thermomixeur à 1500 tours/minute pour une durée de 10 minutes. Ensuite on éclaire le liquide ionique à une longueur d'onde de 365 nm, au moyen de la fibre optique 31 a ou d'une lampe à fluorescence disposée au-dessus de la cavité 1. Le 8-hydroxyquinoline non complexé n'est pas excité par cette longueur d'onde, tandis qu'après complexation on observe une bande de fluorescence centrée à 485 nm.

Jusqu'à présent, seul le cas d'un dispositif isolé a été considéré. En réalité, un des avantages de l'invention réside dans la possibilité d'effectuer en parallèle des analyses sur plusieurs échantillons, par exemple afin d'identifier et de doser plusieurs ions dans une même solution aqueuse ou pour identifier et doser un même ion dans une pluralité de solutions aqueuses différentes. De telles analyses en parallèle peuvent être effectuées à l'aide du système représenté sur la figure 6, constitué par un agencement d'une pluralité de dispositifs selon l'invention. Ce système comporte un substrat 10' sur lequel ont été réalisés plusieurs systèmes d'électrodes, du type décrit en référence aux figures 3A et 3B, disposés selon une grille régulière ; le substrat 10' constitue le fond de toutes les cavités du système. Les parois latérales 1 a sont réalisées par des ouvertures, disposées selon le même motif en grille des électrodes, pratiquées dans une plaque 12 (plaque à puits), typiquement en plastique, qui est collée au substrat 10'. Le substrat 10' déborde légèrement de la plaque 12 de manière à exposer les contacts électriques 100 qui permettent de relier les électrodes à des dispositifs de mesure. De cette manière, on obtient une « puce » électrochimique qui réunit, en un volume très limité, des dizaines voire des centaines de dispositifs selon l'invention. A titre d'exemple, une plaque à puits présente typiquement 96 ou 384 ouvertures.

La « puce » ainsi réalisée est rendue adaptée à une utilisation particulière en introduisant dans chaque cavité une quantité prédéterminée d'un liquide ionique matrice contenant un liquide ionique à tâche spécifique ou une molécule complexante. Par exemple, si on veut détecter et/ou doser plusieurs types d'ions dans un échantillon d'une même solution (par exemple, tous les ions de métaux lourds toxiques dans un échantillon d'eau potable), chaque cavité contiendra un liquide ionique différent, ou le même liquide ionique avec une molécule ou fonction complexante différente. Au contraire, si on veut effectuer la même analyse sur plusieurs échantillons différents (par exemple, mesurer la teneur en ions $Hg^{2+}$ d'échantillons d'eau prélevés dans des rivières différentes, ou à plusieurs sites le long d'une rivière), le même liquide ionique avec la même molécule complexante sera introduit dans chaque cavité.

On comprend que les différentes analyses peuvent être effectuées en parallèle suivant la méthode illustrée par les figures 2A - 2E, et cela ne requiert pas plus de manipulations qu'une analyse individuelle.

L'invention a été décrite en référence à certains modes de réalisation particuliers, mais des nombreuses variantes sont possibles.

Les ions détectés et dosés peuvent être autres que des ions de métaux lourds. Par exemple, l'invention peut permettre le dosage d'ions de métaux alcalins et alcalino-terreux dans des échantillons biologiques pour effectuer des analyses médicales, de cations ammonium, d'anions d'halogénures, etc.

Des liquides ioniques et des molécules ou fonctions compléxantes ont été cités dans la description, mais uniquement à titre d'exemples non limitatifs. Il en va de même pour les sondes et pour les moyens d'analyse d'ions.

Des solutions à base de solvants autres que l'eau peuvent également être analysées conformément à la présente invention : il suffit que le liquide ionique soit choisi de manière à être immiscible avec ledit solvant et à pouvoir extraire efficacement les ions à doser. La différence de densité entre le liquide ionique et la solution à analyser est également importante afin d'assurer le contact entre ledit liquide ionique et le moyen d'analyse d'ions. Bien que seul le cas d'un liquide ionique plus dense que la solution ait été considéré dans les exemples, l'inverse est également possible ; dans ce cas le moyen d'analyse doit être agencé dans la partie supérieure de la cavité.

Dans l'exemple de réalisation décrit en détail en référence aux figures 3A et 3B, l'agitation du contenu de la cavité a été obtenue à l'aide d'un thermomixeur « macroscopique ». D'autres techniques d'agitation peuvent être considérées. En particulier, il est connu d'agiter des quantités de liquides à l'échelle du microlitre sur la surface d'une puce microfluidique en utilisant des ondes acoustiques de surface. Un dispositif ou système selon l'invention peut comprendre des moyens intégrés d'agitation, basés par exemple sur la technique des ondes acoustiques de surface, ce qui élimine la nécessité d'utiliser un moyen « macroscopique » d'agitation.

Enfin, un dispositif ou système selon l'invention peut ne constituer qu'un élément d'un système de microanalyse plus important, par exemple d'un système microfluidique comportant des moyens pour déplacer des faibles quantités de liquides sur une puce.

## Revendications

1. Procédé de microanalyse d'ions, comportant les étapes suivantes:

   - disposer, à l'intérieur d'une cavité (1) ayant un volume interne, un volume d'un liquide ionique (2) qui est inférieur audit volume interne ;
   - disposer à l'intérieur de ladite cavité (1) une solution (4) contenant lesdits ions ($C^+$, $A^-$) à analyser, le solvant de la solution (4) et le liquide ionique (2) étant choisis de manière à être immiscibles et à permettre un transfert des ions ($C^+$, $A^-$) de ladite solution (4) vers ledit liquide ionique (2) ; et le procédé étant **caractérisé en ce qu'**il comporte l'étape suivante:
   - détecter la présence desdits ions ($C^+$, $A^-$) dans ledit liquide ionique (2) à l'aide d'un moyen d'analyse (3) d'au moins un type desdits ions en solution dans ledit liquide ionique (2), à l'état libre ou de complexe, ledit moyen d'analyse (3) étant du type moyen d'analyse éléctrochimique ou moyen d'analyse optique par spectrophotométrie ou luminescence, et comprenant au moins un moyen de détection disposé au contact dudit liquide ionique (2).

2. Procédé selon la revendication 1 **caractérisé en ce qu'**il comporte également une étape d'agitation dudit liquide ionique (2) et de ladite solution (4) de manière à favoriser ledit transfert des ions (C$^+$, A$^-$).

3. Procédé selon l'une des revendications précédentes, **caractérisé en ce que** ladite étape consistant à détecter la présence desdits ions (C$^+$, A$^-$) dans ledit liquide ionique (2) à l'aide dudit moyen d'analyse (2) comporte une mesure quantitative de la concentration desdits ions (C$^+$, A$^-$).

4. Procédé selon l'une des revendications précédentes, **caractérisé en ce que** le liquide ionique (2) contient en solution au moins une molécule ayant une fonction de complexation dudit type d'ions (C$^+$, A$^-$) à extraire et analyser.

5. Procédé selon l'une des revendications 1 à 3, **caractérisé en ce que** le liquide ionique (2) est mélangé avec un liquide ionique à tâche spécifique dont au moins un anion ou un cation porte une fonction de complexation dudit type d'ions (C$^+$, A$^-$) à extraire et analyser.

6. Procédé selon l'une des revendications précédentes, **caractérisé en ce que** ledit liquide ionique (2) ou ladite molécule ayant une fonction de complexation comprennent une fonction sonde dont au moins une propriété physique ou chimique détectable varie en réponse à la complexation des ions à analyser.

7. Procédé selon l'une des revendications précédentes, **caractérisé en ce que** ledit volume de liquide ionique (2) est inférieur au volume de la solution (4) contenant les ions à analyser (C$^+$, A$^-$), de manière à permettre une extraction concentrante desdits ions (C$^+$, A$^-$).

8. Procédé selon l'une des revendications précédentes, **caractérisé en ce que** ledit moyen d'analyse (3) d'au moins un type d'ions en solution dans ledit liquide ionique (2) est un moyen d'analyse électrochimique (30) comprenant au moins deux et de préférence trois électrodes (30a, 30b, 30c) en contact avec ledit volume de liquide ionique (2), ladite étape consistant à détecter la présence desdits ions (C$^+$, A$^-$) dans ledit liquide ionique (2) à l'aide dudit moyen d'analyse (3) étant effectuée au moyen d'une technique électrochimique.

9. Procédé selon la revendication 8, **caractérisé en ce que** ladite technique électrochimique est une mesure par voltampérométrie pulsée différentielle ou par voltampérométrie cyclique.

10. Procédé selon l'une des revendications 1 à 7, **caractérisé en ce que** ledit moyen d'analyse (3) d'au moins un type d'ions en solution dans ledit liquide ionique (2) est un moyen d'analyse optique par spectrophotométrie ou luminescence, ladite étape consistant à détecter la présence desdits ions (C$^+$, A$^-$) dans ledit liquide ionique (2) à l'aide dudit moyen d'analyse (3) étant effectuée au moyen d'une technique optique.

11. Dispositif adapté pour la mise en oeuvre d'un procédé de microanalyse d'ions selon l'une quelconque des revendications précédentes, comprenant:

- une cavité (1) ayant un volume interne ;
- un volume d'un liquide ionique (2) disposé à l'intérieur de ladite cavité (1), ledit volume de liquide ionique (2) étant inférieur au volume interne de la cavité (1) ; et **caractérisé par**
- un moyen d'analyse (3) d'au moins un type d'ions (C$^+$, A$^-$) en solution dans ledit liquide ionique (2) à l'état libre ou de complexe, ledit moyen d'analyse (3) étant du type moyen d'analyse électrochimique ou moyen d'analyse optique par spectrophotométrie ou luminescence, et comprenant au moins un moyen de détection disposé à l'intérieur de ladite cavité (1), en contact avec ledit volume de liquide ionique (2).

12. Dispositif selon la revendication 11, **caractérisé en ce que** ladite cavité présente un fond (1b) constitué par un substrat de circuit intégré (10, 10') sur lequel sont formées au moins deux, et de préférence au moins trois, électrodes (30a, 30b, 30c) en contact avec ledit volume de liquide ionique et constituant un moyen d'analyse électrochimique (30) d'au moins un type d'ions en solution dans ledit liquide ionique.

13. Dispositif selon la revendication 12, **caractérisé en ce que** ladite cavité présente une paroi latérale (1a) formée par un puits d'une plaque à puits (14) fixée de manière étanche audit substrat de circuit intégré (10').

14. Système pour la microanalyse d'ions, **caractérisé en ce qu'**il comporte une pluralité de dispositifs selon l'une des revendications 11 à 13, chacun desdits dispositif contenant un liquide ionique (2) pour l'extraction sélective d'un type d'ions différent.

15. Système pour la microanalyse d'ions, **caractérisé en ce qu'**il comporte une pluralité de dispositifs selon l'une des revendications 11 à 13, chacun desdits dispositif contenant un liquide ionique (2) pour l'extraction sélective d'un même type d'ions.

**Patentansprüche**

1. Verfahren zur Mikroanalyse von Ionen, umfassend die folgenden Schritte:

   - Deponieren, im Inneren eines Hohlraums (1) mit einem Innenvolumen, ein Volumen einer ionischen Flüssigkeit (2), welches kleiner als das Innenvolumen ist;
   - Deponieren im Inneren des Hohlraums (1) einer Lösung (4), welche zu analysierende Ionen ($C^+$, $A^-$) enthält, wobei das Lösungsmittel der Lösung (4) und die ionische Flüssigkeit (2) derart ausgewählt sind, dass sie unvermischbar sind und dass ein Transfer von Ionen ($C^+$, $A^-$) der Lösung (4) in Richtung der ionischen Flüssigkeit (2) ermöglicht wird; und wobei das Verfahren **dadurch gekennzeichnet ist, dass** es den folgenden Schritt umfasst:
   - Erfassen des Vorhandenseins der Ionen ($C^+$, $A^-$) in der ionischen Flüssigkeit (2) mit Hilfe eines Mittels zur Analyse (3) wenigstens eines Typs der Ionen, welche in der ionischen Flüssigkeit (2) gelöst sind, im freien Zustand oder Zustand eines Komplexes, wobei das Mittel zur Analyse (3) von dem Typ eines Mittels zur elektrochemischen Analyse oder eines Mittels zur optischen Analyse durch Spektrophotometrie oder Lumineszenz ist und wenigstens ein Erfassungsmittel im Kontakt mit der ionischen Flüssigkeit (2) umfasst.

2. Verfahren nach Anspruch 1, **dadurch gekennzeichnet, dass** es außerdem einen Schritt eines Rührens der ionischen Flüssigkeit (2) und der Lösung (4), sodass der Transfer der Ionen ($C^+$, $A^-$) befördert wird, umfasst.

3. Verfahren nach einem der vorhergehenden Ansprüche, **dadurch gekennzeichnet, dass** der Schritt, welcher darin besteht, das Vorhandensein der Ionen ($C^+$, $A^-$) in der ionischen Flüssigkeit (2) mit Hilfe des Mittels zur Analyse (2) zu Erfassen, ein quantitatives Messen der Konzentration der Ionen ($C^+$, $A^-$) umfasst.

4. Verfahren nach einem der vorhergehenden Ansprüche, **dadurch gekennzeichnet, dass** die ionische Flüssigkeit (2) in Lösung wenigstens ein Molekül enthält, welches eine Funktion einer Komplexierung des zu extrahierenden und analysierenden Typs von Ionen ($C^+$, $A^-$) aufweist.

5. Verfahren nach einem der Ansprüche 1-3, **dadurch gekennzeichnet, dass** die ionische Flüssigkeit (2) mit einer ionischen Flüssigkeit mit spezifischer Aufgabe gemischt ist, von welcher wenigstens ein Anion oder ein Kation eine Funktion einer Komplexierung des zu extrahierenden und analysierenden Typs von Ionen ($C^+$, $A^-$) trägt.

6. Verfahren nach einem der vorhergehenden Ansprüche, **dadurch gekennzeichnet, dass** die ionische Flüssigkeit (2) oder dass eine Funktion einer Komplexion aufweisende Molekül eine Sondenfunktion umfassen, von welcher wenigstens eine erfassbare physikalische oder chemische Eigenschaft in Reaktion auf die Komplexierung der zu analysierenden Ionen variiert.

7. Verfahren nach einem der vorhergehenden Ansprüche, **dadurch gekennzeichnet, dass** das Volumen der ionischen Flüssigkeit (2) kleiner als das Volumen der die zu analysierenden Ionen ($C^+$, $A^-$) enthaltenden Lösung (4) ist, sodass eine konzentrierende Extrahierung der Ionen ($C^+$, $A^-$) ermöglicht wird.

8. Verfahren nach einem der vorhergehenden Ansprüche, **dadurch gekennzeichnet, dass** Mittel zur Analyse (3) des wenigstens einen Typs von Ionen in Lösung in der ionischen Flüssigkeit (2) ein Mittel zur elektrochemischen Analyse (3) ist, welches wenigstens zwei und bevorzugt drei Elektroden (30a, 30b, 30c) in Kontakt mit dem Volumen der ionischen Flüssigkeit (2) umfasst, wobei der Schritt, welcher darin besteht, das Vorhandensein der Ionen ($C^+$, $A^-$) in der ionischen Flüssigkeit (2) mit Hilfe des Mittels zur Analyse (3) zu erfassen, mittels einer elektrochemischen Technik bewerkstelligt wird.

9. Verfahren nach Anspruch 8, **dadurch gekennzeichnet, dass** die elektrochemische Technik eine Messung durch gepulste Voltammetrie ist.

**10.** Verfahren nach einem der Ansprüche 1-7, **dadurch gekennzeichnet, dass** das Mittel zur Analyse (3) des wenigstens einen Typs von Ionen in Lösung in der ionischen Flüssigkeit (2) ein Mittel zur optischen Analyse durch Spektrophotometrie oder Lumineszenz ist, wobei der Schritt, welcher darin besteht, das Vorhandensein der Ionen (C$^+$, A$^-$) in der ionischen Flüssigkeit (2) mit Hilfe des Mittels zur Analyse (3) zu erfassen, mittels einer optischen Technik bewerkstelligt wird.

**11.** Vorrichtung, welche ausgestaltet ist für den Einsatz eines Verfahrens zur Mikroanalyse von Ionen nach einem beliebigen der vorhergehenden Ansprüche, umfassend:

   - einen Hohlraums (1) mit einem Innenvolumen;
   - ein Volumen einer ionischen Flüssigkeit (2), welches im Inneren des Hohlraums (1) deponiert ist, wobei das Volumen der ionischen Flüssigkeit (2) kleiner ist als das Innenvolumen des Hohlraums (1); und **gekennzeichnet durch**
   - ein Mittel zur Analyse (3) wenigstens eines Typs von Ionen (C$^+$, A$^-$) in Lösung in der ionischen Flüssigkeit (2), im freien Zustand oder Zustand eines Komplexes, wobei das Mittel zur Analyse (3) von dem Typ eines Mittels zur elektrochemischen Analyse oder eines Mittels zur optischen Analyse **durch** Spektrophotometrie oder Lumineszenz ist und wenigstens ein Erfassungsmittel umfasst, welches im Inneren des Hohlraums (1) im Kontakt mit dem Volumen von ionischer Flüssigkeit (2) angeordnet ist.

**12.** Vorrichtung nach Anspruch 11, **dadurch gekennzeichnet, dass** der Hohlraum einen Boden (1b) aufweist, welcher gebildet ist durch ein Substrat einer integrierten Schaltung (10, 10'), auf welchem wenigstens zwei, und bevorzugt wenigstens drei Elektroden (30a, 30b, 30c) im Kontakt mit dem Volumen von ionischer Flüssigkeit ausgebildet sind und ein Mittel zur elektrochemischen Analyse (30) wenigstens eines Typs von Ionen in Lösung in der ionischen Flüssigkeit bilden.

**13.** Vorrichtung nach Anspruch 12, **dadurch gekennzeichnet, dass** der Hohlraum eine Seitenwand (1a) aufweist, welche gebildet ist durch einen Well einer Multiwellplatte (14), welcher auf abdichtende Weise an dem Substrat der integrierten Schaltung (10') befestigt ist.

**14.** System für die Mikroanalyse von Ionen, **dadurch gekennzeichnet, dass** es eine Vielzahl von Vorrichtungen nach einem der Ansprüche 11-13 umfasst, wobei jede dieser Vorrichtungen eine ionische Flüssigkeit (2) für das selektive Extrahieren eines anderen Typs von Ionen enthält.

**15.** System für die Mikroanalyse von Ionen, **dadurch gekennzeichnet, dass** es eine Vielzahl von Vorrichtungen nach einem der Ansprüche 11-13 umfasst, wobei jede dieser Vorrichtungen eine ionische Flüssigkeit (2) für das selektive Extrahieren eines gleichen Typs von Ionen enthält.

**Claims**

**1.** A method of microanalyzing ions, comprising the following steps:

   • placing in a cavity (1) having an internal volume, a volume of an ionic liquid (2) that is smaller than said internal volume;
   • placing in said cavity (1) a solution (4) containing said ions (C$^+$, A$^-$) that are to be analyzed, the solvent of the solution (4) and the ionic liquid (2) being selected so as to be immiscible and so as to enable ions (C$^+$, A$^-$) to be transferred from said solution (4) to said ionic liquid (2); and the method being **characterized in that** it comprises the following step:
   • detecting the presence of said ions (C$^+$, A$^-$) in said ionic liquid (2) with analyzer means (3) for analyzing at least one type of said ions in solution in said ionic liquid (2), in the free state or in the complexed state, said analyzer means (3) placed in contact with said ionic liquid (2) being of the type electrochemical analyzer means or optical analyzer means using spectrophotometry or luminescence, and comprising at least one detection means placed in contact with said ionic liquid (2).

**2.** A method according to claim 1, **characterized in that** it also includes a step of stirring said ionic liquid (2) and said solution (4) in such a manner as to facilitate said transfer of ions (C$^+$, A$^-$).

**3.** A method according to any preceding claim, **characterized in that** said step that consists in detecting the presence of said ions (C⁺, A⁻) in said ionic liquid (2) with said analyzer means (2) comprises quantitative measurement of the concentration of said ions (C⁺, A⁻).

4.  A method according to any preceding claim, **characterized in that** said ionic liquid (2) contains in solution at least one molecule having a completing function for said type of ions (C⁺, A") to be extracted and analyzed.

5.  A method according to any one of claims 1 to 3, **characterized in that** the ionic liquid (2) is mixed with a task-specific ionic liquid having at least one anion or cation that carries a complexing function for said type of ions (C⁺, A⁻) to be extracted and analyzed.

6.  A method according to any preceding claim, **characterized in that** said ionic liquid (2) or said molecule having a complexing function include a probe function having at least one detectable physical or chemical property that varies in response to the complexing of the ions to be analyzed.

7.  A method according to any preceding claim, **characterized in that** said volume of ionic liquid (2) is less that the volume of the solution (4) containing the ions to be analyzed (C⁺, A⁻), so as to enable said ions (C⁺, A⁻) to be concentrated on being extracted.

8.  A method according to any preceding claim, **characterized in that** said analyzer means (3) for analyzing at least one type of ion in solution in said ionic liquid (2) comprise electrochemical analyzer means (30) having at least two and preferably three electrodes (30a, 30b, 30c) in contact with said volume of ionic liquid (2), said step that consists in detecting the presence of said ions (C⁺, A⁻) in said ionic liquid (2) with said analyzer means (3) being performed by using an electrochemical technique.

9.  A method according to claim 8, **characterized in that** said electrochemical technique is by differential pulse volt-amp measurement or by cyclical volt-amp measurement.

10. A method according to any one of claims 1 to 7, **characterized in that** said analyzer means (3) for at least one type of ion in solution in said ionic liquid (2) are optical analyzer means using spectrophotometry or luminescence, said step that consists in detecting the presence of said ions (C⁺, A⁻) in said ionic liquid (2) with said analyzer means (3) being performed by means of an optical technique.

11. A device adapted for implementing a method of microanalyzing ions in accordance with any preceding claim, the device comprising:

    • a cavity (1) having an internal volume;
    • a volume of an ionic liquid (2) placed inside said cavity (1), said volume of ionic liquid (2) being less than the inside volume of said cavity (1); and **characterized by**:
    • analyzer means (3) for analyzing at least one type of ion (C⁺, A⁻) in solution in said ionic liquid (2) in the free or the complexed state, said analyzer means (3) being of the type electrochemical analyzer means or optical analyzer means using spectrophotometry or luminescence, and comprising at least one detection means disposed inside said cavity (1) in contact with said volume of ionic liquid (2).

12. A device according to claim 11, **characterized in that** said cavity presents a bottom (1b) constituted by an integrated circuit substrate (10, 10') on which there are formed at least two and preferably at least three electrodes (30a, 30b, 30c) in contact with said volume of ionic liquid and constituting means (30) for electrochemically analyzing at least one type of ion in solution in said ionic liquid.

13. A device according to claim 12, **characterized in that** said cavity presents a side wall (1a) formed by a well in a well plate (14) fastened in leaktight manner to said integrated circuit substrate (10').

14. A system for microanalyzing ions, the system being **characterized in that** it comprises a plurality of devices according to any one of claims 11 to 13, each of said devices containing an ionic liquid (2) for selective extraction of ions of different types.

15. A system for microanalyzing ions, the system being **characterized in that** it comprises a plurality of devices according to any one of claims 11 to 13, each of said devices containing an ionic liquid (2) for selectively extracting ions of a single type.

FIG.1

FIG.2A

FIG.2B

FIG.2C

FIG.2D

FIG.2E

FIG.3a

FIG.3b

FIG.4

FIG.5

FIG.6

## RÉFÉRENCES CITÉES DANS LA DESCRIPTION

**Littérature non-brevet citée dans la description**

- **MANABU TOKESHI et al.** Continuous-Flow Chemical Processing on a Microchip by Combining Microunit Opérations and a Multiphase Flow Network. *Anal. Chem.,* 2002, vol. 74, 1565-1571
- **SHENG DAI et al.** Solvent extraction of strontium nitrate by a crown ether using room-temperature ionic liquids. *J. Chem. Soc. Dalton Trans.,* 1999, 1201-1202
- **HUIMIN LUO et al.** Extraction of Cesium Ions from Aqueous Solutions Using Calix[4]arene-bis(tert-octylbenzo-crown-6) in Ionic Liquids. *Anal. Chem.,* 2004, vol. 76, 3078-3083
- **JING-FU LIU et al.** Ionic liquid-based liquid-phase microextraction, a new sample enrichment procédure for liquid chromatography. *Journal of Chromatography A,* 2004, vol. 1026, 143-147
- Non-Holo aluminat Room-Temperature Ionic Liquids in Electrochemistry - A Review. **M.BUZZEO et al.** CHEM PHYSCHEM. Wiley, vol. 5
- **CABALLERO et al.** *J. Am. Chem. Soc.,* 2005, vol. 127 (45), 15666-15667